(19) Europäisches Patentamt
European Patent Office
Office européen des brevets

(11) Numéro de publication: **0 136 195**
**B1**

(12) # FASCICULE DE BREVET EUROPEEN

(45) Date de publication du fascicule du brevet: **24.06.87**

(51) Int. Cl.⁴: **C 07 C 123/00,** A 61 K 31/155, A 01 N 37/52

(21) Numéro de dépôt: **84401494.4**

(22) Date de dépôt: **13.07.84**

(54) **Dérivés de la benzamidine à activité antimicrobienne, procédé d'obtention et application à titre de médicaments désinfectants ou conservateurs.**

(30) Priorité: **03.08.83 FR 8312824**

(43) Date de publication de la demande:
**03.04.85 Bulletin 85/14**

(45) Mention de la délivrance du brevet:
**24.06.87 Bulletin 87/26**

(84) Etats contractants désignés:
**AT BE CH DE FR GB IT LI LU NL SE**

(56) Documents cités:
**EP - A - 0 076 430**

**PHARMAZIE, vol. 30, no. 6, juin 1975, pages 386-389, Berlin, DE; P. WALSMANN et al.: "Synthetische Inhibitoren der Serinproteinasen"**

(73) Titulaire: **SANOFI, 40, Avenue George V, F-75008 Paris (FR)**

(72) Inventeur: **Mosse, Madeleine, La Chanterelle Chemin du Réservoir de Montmaur, F-34100 Montpellier (FR)**
Inventeur: **Demarne, Henri, Le Florence Avenue Major Flandre, F-34000 Montpellier (FR)**
Inventeur: **Filhol, Robert, Château d'Alco Bâtiment 7 Rue des Avants-Monts, F-34100 Montpellier (FR)**

(74) Mandataire: **Gillard, Marie-Louise et al, Cabinet Beau de Loménie 55, Rue d'Amsterdam, F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

## Description

La présente invention concerne des nouveaux dérivés de la benzamidine répondant à la formule générale suivante:

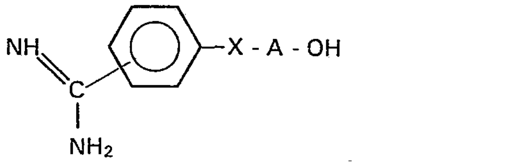

(I)

dans laquelle:

A représente une chaîne alkyle droite ou ramifiée comprenant de 3 à 9 atomes de carbone;

X représente l'atome d'oxygène ou une liaison directe.

Lorsque X représente une liaison directe, la benzamidine est située en para par rapport au groupement alcanol.

La présente invention comprend également les sels pharmaceutiquement acceptables des composés (I).

Ces composés présentent une activité antimicrobienne, ils peuvent notamment être utilisés comme médicaments antiseptiques à usage humain ou vétérinaire ou comme désinfectants sur des surfaces externes inertes. Ils peuvent également être utilisés comme conservateurs pour des produits à utilisation externe.

Les composés selon l'invention sont préparés à partir des nitrophénylalcanols ou des nitrophénoxyalcanols (IV). Par hydrogénation catalytique, on obtient les dérivés de l'aniline correspondants (V), puis par addition du nitrite de sodium, en milieu acide, les diazonium. L'action du cyanure cuivreux conduit ensuite, par la réaction de Sandmeyer, aux dérivés du benzonitrile (VI). Enfin les amidines (I) sont synthétisées par une réaction connue en elle-même qui comprend la formation intermédiaire d'un iminoester.

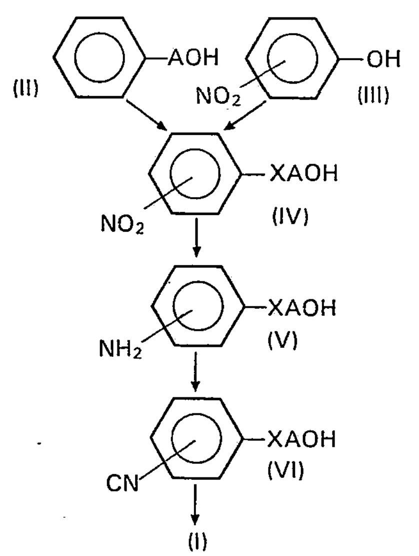

(I)

Lorsque X représente une liaison directe, on prépare le nitrophénylalcanol (IV) à partir du phénylalcanol (II). Au préalable, on protège le groupement hydroxyle par acétylation au moyen du chlorure d'acétyle. Après la nitration par l'acide nitrique fumant, l'alcool est libéré par action du méthanol chlorhydrique.

Les phénylalcanols (II) sont accessibles commercialement lorsqu'il s'agit d'alcools linéaires. Sinon, ils peuvent être préparés par diverses méthodes. Par exemple, les phénylalcools secondaires sont préparés à partir du phénylacétaldéhyde par action d'un dérivé magnésien suivi d'une hydrolyse:

$$C_6H_5 - CH_2 - CHO \xrightarrow[2°/H_2O]{1°/R_1MgBr} C_6H_5 - CH_2 - \underset{\displaystyle OH}{\underset{|}{CH}} - R_1 \quad (II')$$

$R_1$ : alkyle de 1 à 8 carbones.

Les phénylalcanols ramifiés primaires sont obtenus à partir du cyanure de benzyle:

$$C_6H_5 - CH_2 - CN \xrightarrow[2°/R_1Br]{1°/NaH} C_6H_5 - \overset{\displaystyle R_1}{\overset{|}{\underset{\displaystyle R_1}{\underset{|}{C}}}} - CN$$

$$\xrightarrow[2°/H^+]{1°/OH^-,\ glycol} C_6H_5 - \overset{\displaystyle R_1}{\overset{|}{\underset{\displaystyle R_1}{\underset{|}{C}}}} - CO_2H \quad (VII)$$

$$\xrightarrow[2°/C_2H_5OH \ \text{pyridine}]{1°/SOCl_2} C_6H_5 - \overset{\displaystyle R_1}{\overset{|}{\underset{\displaystyle R_1}{\underset{|}{C}}}} - CO_2Et \quad (VIII)$$

$$\xrightarrow{\text{hydrure mixte}} C_6H_5 - \overset{\displaystyle R_1}{\overset{|}{\underset{\displaystyle R_1}{\underset{|}{C}}}} - CH_2OH \quad (II'')$$

Après avoir fait réagir l'hydrure de sodium en milieu anhydre, on peut additionner un halogénure d'alkyle, par exemple le bromure, pour obtenir un phényl-acétonitrile dialkylé symétrique. La transformation de ce composé en acide s'effectue par action d'une base en milieu alcoolique, suivie d'une acidification.

L'action du chlorure de thionyle puis de l'alcool éthylique en milieu anhydre en présence d'un catalyseur, par exemple la pyridine ou la diméthylaminopyridine, permet d'obtenir l'ester éthylique (VII). On prépare ensuite l'alcool correspondant (II'') par réduction à l'acide d'un hydrure mixte dans un solvant anhydre.

Lorsque X représente l'oxygène, on prépare le nitrophénoxyalcanol (IV) à partir du nitrophénol (III). L'action sur (III) d'un dihalogénure d'alkyle en milieu basique permet d'obtenir un halogénure de nitrophénoxyalkyle (IX). Ce produit est acétylé en milieu acide puis (IV) est libéré par saponification.

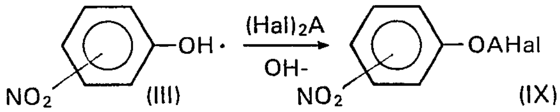


(III) (IX)

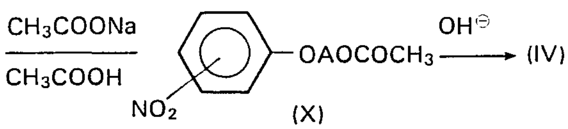


(X) (IV)

Les exemples suivant illustrent l'invention sans toutefois la limiter. Lorsque le produit obtenu est sous forme d'huile, il est caractérisé par son spectre de résonance magnétique nucléaire (RMN). Celui-ci est enregistré à 60 MHz dans le deutérochloroforme, en prenant comme étalon interne l'hexadiméthylsiloxane.

Pour décrire le spectre, les abréviations suivantes sont utilisées:

S: singulet
D: doublet
T: triplet
Q: quadruplet
M: multiplet
J: constante de couplage.

*Exemple 1*

*Chlorhydrate d'(hydroxy-3 propyl)-4 benzamidine:* SR 41326 A.

a) *-(Nitro-4 phényl)-3 propanol-1.*

A 171,5 g de phényl-3 propanol-1, on ajoute en une heure sous agitation, 95 ml de chlorure d'acétyle. On chauffe 2 h à reflux, l'acide chlorhydrique qui se dégage et l'excès de chlorure d'acétyle sont éliminés. Lorsque le milieu réactionnel est revenu à la température ambiante, on le verse goutte à goutte, en agitant, sur 800 ml d'acide nitrique fumant (d = 1,49) refroidi à −25°C, l'addition dure 1 h pendant laquelle la température est maintenue entre −15°C et −20°C. On verse ensuite sur 1,5 l d'eau additionnée de glace pilée puis on extrait 3 fois à l'éther, lave 3 fois à l'eau, 3 fois avec une solution de carbonate de sodium à 10%, puis 3 fois à l'eau. Les phases éthérées sont séchées sur sulfate de magnésium puis évaporées à sec sous pression réduite. Le résidu est repris dans 800 ml de méthanol puis on fait barboter de l'acide chlorhydrique gazeux 1 h à 0°C, on chauffe ensuite pendant 14 h à reflux. Après évaporation du solvant, on reprend dans le mélange eau-éther, décante la phase acqueuse, lave 2 fois à l'eau, 3 fois avec une solution saturée de bicarbonate de sodium puis 3 fois à l'eau; la phase éthérée est ensuite séchée sur sulfate de magnésium, puis evaporée à sec sous pression réduite.

On obtient 259 g d'une huile orangée que l'on purifie par chromatographie sur 3 kg de gel de silice dans le chloroforme; on récupère 218 g d'huile orangée; Rendement 95%.

b) *-(hydroxy-3 propyl)-4, aniline.*

218 g de (nitro-4 phényl)-3 propanol-1 sont dissous dans 500 ml de méthanol, on ajoute 10 g de palladium à 10% sur charbon préalablement humectés avec 10 ml d'eau.

L'hydrogénation s'effectue sous une pression de 40 bars et sous agitation, elle dure 1 h 30. On filtre ensuite sur Célite, rince au méthanol, évapore à sec sous pression réduite pour obtenir 168 g d'une huile marron. Celle-ci est purifiée par 3 chromatographies successives sur 6 kg d'alumine au total, en utilisant le dichlorométhane comme éluant. On obtient 49,2 g d'une poudre marron clair; point de fusion (Fc) = 43-45°C; Rendement 27%.

c) *-(hydroxy-3 propyl)-4 benzonitrile.*

49,07 g du produit obtenu précédemment sont versés dans 87 ml d'acide chlorhydrique concentré additionné de 400 g de glace pilée. En maintenant la température entre 0°C et 5°C, on ajoute goutte à goutte une solution de 23,15 g de nitrite de sodium dans 80 ml d'eau puis, après 10 min d'agitation, on neutralise avec 300 ml de solution à 10% de carbonate de sodium.

On prépare par ailleurs une solution de cyanure cuivreux: 40,35 g de chlorure cuivreux sont mis en suspension dans 150 ml d'eau, on ajoute une solution de 54 g de cyanure de sodium dans 80 ml d'eau. On observe un dégagement de chaleur, le chlorure cuivreux se dissout, la solution est décolorée. A cette solution refroidie à 0°C et additionnée de 200 ml de benzène, on ajoute goutte à goutte pendant 40 min en agitant violemment la solution de diazonium refroidie à 0°C. Après 40 min d'agitation supplémentaire, on laisse revenir à température ambiante, sous agitation, puis on chauffe à 50°C sans agiter et on ramène à température ambiante.

On extrait 3 fois à l'éther, lave 2 fois à l'eau, puis avec une solution saturée de chlorure de sodium. Les phases éthérées sont séchées sur sulfate de magnésium et évaporées à sec sous pression réduite. On obtient 51 g d'une huile marron foncé. Celle-ci est purifiée par chromatographie sur 1 500 g de sel de silice, la colonne est préparée dans du toluène et l'éluant est un mélange toluène-éther (9/l en volume). On obtient 41,6 g de produit pur sous forme d'huile rouge; Rendement 79%.

Le produit est caractérisé par son spectre de RMN:

2H entre 1,7 et 2,2 ppm (M, $-CH_2-\underline{CH_2}-CH_2-OH$)
1H à 2,4 ppm (S, -OH)
2H à 2,8 ppm (T, J = 7 Hz, $CN-C_6H_4-\underline{CH_2}-CH_2$)
2H à 3,6 ppm (T, J = 6 Hz, $-CH_2-\underline{CH_2}-OH$)
2H à 7,3 ppm (D, J = 9 Hz, $\underline{H}$ ortho $CH_2$)
2H à 7,6 ppm (D, J = 9 Hz, $\underline{H}$ ortho CN)

d) *-chlorhydrate d'(hydroxy-3 propyl)-4 phényl formimidate d'éthyle.*

30,6 g du produit obtenu précédemment sont dissous dans 300 ml d'alcool absolu, on fait barboter l'acide chlorhydrique gazeux à 0°C pendant 5 h puis on laisse reposer 24 h à température ambiante, les

solvants sont evaporés à sec sous pression réduite et le résidu est précipité dans 1 litre d'éther. On laisse sous agitation pendant 1 h à 0°C puis on filtre, lave à l'éther et sèche sous vide au dessiccateur sur anhydride phosphorique. On obtient 40 g d'une poudre rose; Fc = 104-105°C avec décomposition; Rendement 86%.

e) *- SR 41326 A.*

40 g du produit précédemment obtenu sont dissous dans 400 ml d'alcool absolu. On fait barboter de l'ammoniac gazeux à 0°C pendant 5 h, puis on laisse à température ambiante sous agitation et on abandonne 48 h au repos. Les solvants sont ensuite évaporés à sec sous pression réduite et le résidu est repris dans 500 ml d'eau distillée. On agite 1 h avec 450 ml de résine Amberlite IRA 400 sous forme acétate puis on filtre et lave 3 fois par 300 ml d'eau. Les filtrats sont ensuite agités avec 400 ml de résine Bio-Rex 70 sous forme acide puis on filtre et lave 3 fois par 500 ml d'eau.

L'élution de l'amidine de la résine est effectuée par des fractions de 500 ml d'acide chlorhydrique à 5% dans l'eau. On évapore ensuite le solvant sous pression réduite, on obtient des cristaux jaunes que l'on reprend dans l'alcool puis on évapore l'alcool (3 fois). Le résidu est repris dans 1 l d'éther et trituré à froid, on obtient une poudre jaune que l'on filtre, lave à l'éther et sèche sous vide au dessiccateur sur anhydride phosphorique. On obtient 20,1 g du produit attendu sous forme de poudre jaune clair; Fc = 195-205°C; Rendement 57%.

*Exemple 2*

*Chlorhydrate d'(hydroxy-2 butyl)-4 benzamidine:* CM 41092 A.

a) *- phényl-1 butanol-2.*

A 2,92 g de magnésium en tournures, on ajoute sous azote, goutte à goutte, à une vitesse suffisante pour maintenir un léger reflux, une solution de 7,5 ml de bromure d'éthyle dans 50 ml d'éther anhydre. Toujours sous azote, on agite pendant 2 h à température ambiante, puis on ajoute goutte à goutte 9,4 ml de phénylacétaldéhyde et on laisse 2 h à température ambiante sous agitation.

On décompose ensuite sur 200 ml de chlorure d'ammonium à 20% refroidi à 0°C et on extrait 3 fois à l'éther. Après 3 lavages à l'eau, les phases éthérées sont séchées sur sulfate de magnésium et évaporées à sec sous pression réduite. On obtient 12,2 g d'une huile légèrement jaune.

b) *-(hydroxy-2 butyl)-4 benzonitrile.*

En opérant comme dans l'exemple 1 a) on prépare ensuite le (nitro-4 phényl)-1 butanol-2 caractérisé par son spectre de RMN:

3H à 0,9 ppm (T. asymétrique, j = 7 Hz, $-CH_3$)
3H entre 1,2 et 1,8 ppm [massif, $-CH(OH)-CH_2-CH_3$]
2H entre 2,6 et 2,9 ppm (M, $CN-C_6H_4-CH_2$)
1H entre 3,4 et 3,9 ppm [M, $CH_2-CH(OH)-CH_2$]
2H à 7,3 ppm (D, J = 9 Hz, H ortho $CH_2$)
2H à 8,1 ppm (D, J = 9 Hz, H ortho CN)

Puis on prépare, comme dans l'exemple 1 b) et c), l'hydroxy-1 butyl)-4 benzonitrile également caractérisé par son spectre de RMN:

3H à 0,8 ppm (T. asymétrique, J = 6 Hz, $-CH_2-CH_3$)
2H à 1,25 ppm [Q, J = 6 Hz, $-CH(OH)-CH_2-CH_3$]
2H entre 2,5 et 2,8 ppm (M, $CN-C_6H_4-CH_2$)
1H entre 3,2 et 3,8 ppm [M, $CH_2-CH(OH)-CH_2$]
1H à 4,5 ppm (D, J = 6 Hz, -OH)
2H à 7,4 ppm (D, J = 9 Hz, H ortho $CH_2$)
2H à 7,7 ppm (D, J = 9 Hz, H ortho CN).

c) *-chlorhydrate d'(hydroxy-2 butyl)-4 formimidate d'éthyle.*

Préparé selon le mode opératoire décrit à l'exemple 1 d), ce produit est recristallisé dans un mélange éthanol-éther; Fc = 118-122°C avec décomposition.

d) *-CM 41092 A.*

Le produit attendu est préparé selon l'exemple 1 e); Fc = 159-161°C, solvant de récristallisation: éthanol-éther.

*Exemple 3*

*Chlorhydrate d'(hydroxy-2 di n-propyl-1,1 éthyl)-4 benzamidine:* SR 41946 A.

a) *- di n-propyl-1,1 phényl acétonitrile.*

On prépare une suspension de 52,4 g d'hydrure de sodium dans 250 ml de diméthylformamide. Sous azote et en maintenant une agitation mécanique, on ajoute 47 ml de cyanure de benzyle, après 35 min, on refroidit dans un bain de glace puis on ajoute très lentement 145 ml de bromure de n-propyle. On maintient ensuite l'agitation pendant 3 h à température ambiante.

On verse sur 2 l d'eau mélangée à de la glace puis on extrait 2 fois à l'éther, lave 3 fois à l'eau et sèche les phases éthérées sur sulfate de magnésium après évaporation de l'éther. Le résidu est distillé sous vide; Eb = 72-80°C sous 0,01 mm Hg. On obtient 50,4 g d'une huile jaune; Rendement 63%.

b) *-acide 1,1 di n-propyl phényl acétique.*

Dans 65 ml de glycol, on dissout 25,1 g du produit obtenu précédemment et on ajoute 15,5 g de potasse en pastilles. En chauffant sous agitation, on distille tout d'abord 2 ml d'eau, puis on maintient le chauffage à reflux sous agitation pendant 40 h et on verse 1,5 l d'eau additionnée de glace. On extrait 2 fois à l'hexane, filtre la phase aqueuse sur Célite et acidifie à pH 1 avec de l'acide chlorhydrique concentré. Le précipité blanc formé est abandonné 48 h au réfrigérateur, filtré, lavé à l'eau et recristallisé dans un mélange méthanol-eau (50-50 en volume). Les cristaux formés sont filtrés, lavés à l'eau et séchés sous vide.

On obtient 18,75 g de cristaux blancs grisâtres; Fc = 97-100°C; Rendement 68%.

c) *-di n-propyl-1,1 phényl acétate d'éthyle.*

La réaction suivante s'effectue sous azote. A 8,8 g du produit précédent, on ajoute goutte à goutte et sous agitation 10 ml de chlorure de thionyle. On

maintient l'agitation 2 h à température ambiante puis on refroidit et extrait l'excès de chlorure de thionyle sous pression réduite. On ajoute ensuite à 0°C, 50 ml d'alcool absolu et 3,5 ml de pyridine anhydre, après 1 h d'agitation, on chauffe 15 h à reflux, puis on refroidit, évapore l'alcool et reprend dans un mélange eau-éther, on extrait 3 fois à l'éther, lave 2 fois avec une solution saturée de bicarbonate de sodium, 1 fois à l'eau, 2 fois à l'acide chlorhydrique normal, puis 3 fois à l'eau. Les phases éthérées sont ensuite séchées sur sulfate de magnésium et évaporées à sec sous pression réduite.

On obtient m: 9,82 g d'huile orangée; Rendement 99%. Le produit est caractérisé par son spectre de RMN:

13H entre 0,6 et 1,4 ppm [massif, $-CO_2-CH_2-C\underline{H}_3$, $(-CH_2-C\underline{H}_2-C\underline{H}_3)$ 2)]
4H entre 0,8 et 2,2 ppm [massif, $(-C\underline{H}_2-CH_2-CH_3)$ 2)]
2H à 4,1 ppm (Q, J = 7 Hz, $-CO_2-C\underline{H}_2-CH_3$)
5H à 7,3 ppm (S, H aromatique).

d) *-di n-propyl-1,1 phényl éthanol.*

3,2 g d'hydrure d'aluminium-lithium sont mis en suspension dans 50 ml de tétrahydrofuranne anhydre, sous azote. On ajoute goutte à goutte, en maintenant un léger reflux, une solution de 9,8 g du produit obtenu en c) dans 50 ml de tétrahydrofuranne, puis on chauffe 4 h à reflux, toujours sous azote. Après refroidissement, on décompose avec 10 ml d'eau et 100 ml d'acide sulfurique à 15%. On extrait 3 fois à l'éther, lave 3 fois à l'eau, les phases éthérées sont séchées sur sulfate de magnésium et évaporées à sec sous pression réduite.

On obtient 8,30 g d'huile jaune que l'on purifie par chromatographie sur 250 g de gel de silice en utilisant comme éluant un mélange chloroforme-hexane (50-50 en volume). On recueille alors 7,91 g d'huile légèrement jaune; Rendement 97%. Le produit est caractérisé par son spectre de RMN:

15H entre 0,5 et 1,8 ppm (massif, 14H des chaînes aliphatiques + O$\underline{H}$)
2H à 3,7 ppm (S, $-C\underline{H}_2-OH$)
5H à 7,25 ppm (S, $\underline{H}$ aromatiques).

e) *-SR 41946 A.*

On effectue ensuite les étapes de synthèse décrites à l'exemple 1 pour préparer le SR 41946 A; Fc = 110-115°C, solvant de recristallisation; mélange éthanol-éther.

*Exemple 4*

*(hydroxy-4 butyloxy)-4 benzamidine:* CM 40847 A.

a) *-(nitro-4 phénoxy)-1, bromo-4 butane.*

A une solution de nitro-4 phénol dans 275 ml d'eau, on ajoute 83 ml de dibromo-1,4 butane puis, goutte à goutte, 49,5 ml de soude 10N sous agitation. On chauffe à reflux sous agitation pendant 24 h.

Après refroidissement, on extrait 3 fois à l'éther, lave 6 fois à la soude normale, puis 3 fois à l'eau. Les phases éthérées sont séchées sur sulfate de sodium et évaporées, on filtre l'insoluble. Le filtrat est évaporé à sec et le résidu est tiré sous vide (0,05 mm Hg). Après trituration dans l'hexane, on obtient des cristaux que l'on filtre, lave à l'hexane et sèche sous vide au dessiccateur.

On obtient 75 g d'un produit pâteux de couleur crème; Rendement 55%.

b) *-(nitro-4 phénoxy)-1 acétyloxy-4 butane.*

75 g du produit précédent sont dissous dans 80 ml d'acide acétique glacial, on ajoute 45 g d'acétate de sodium anhydre, puis on chauffe à reflux pendant 15 heures sous agitation. Le mélange réactionnel est versé sur 1 l d'eau glacée additionnée de 500 ml d'éther et on neutralise à pH 7,5 par du carbonate de sodium solide. Après 3 extractions à l'éther et 3 lavages à l'eau, les phases éthérées sont séchées sur sulfate de magnésium et évaporées à sec, le résidu est tiré sous vide.

On obtient 70 g d'une huile orangée; Rendement 100%.

c) *-(nitro-4 phénoxy)-1 butanol-4.*

70 g du produit obtenu précédemment sont mis en solution dans 300 ml de méthanol, on ajoute 30 ml de soude 10N puis on chauffe à reflux pendant 4 heures sous agitation. Après avoir évaporé le méthanol, le résidu est repris dans un mélange eau-éther, on extrait 3 fois à l'éther, lave 3 fois avec une solution saturée de chlorure de sodium.

Les phases éthérées sont ensuite séchées sur sulfate de magnésium et évaporées à sec. Les cristaux formés sont triturés dans l'hexane, filtrés, lavés à l'hexane et séchés sous vide au dessiccateur.

On obtient 48,8 g de cristaux légèrement jaunes; Fc = 53-55°C.

d) *-CM 40847.*

En opérant comme dans l'exemple 1, on prépare ensuite les composés suivants:
— (hydroxy-4 butyloxy)-4 aniline.
Fc = 56-58°C.
— (hydroxy-4 butyloxy)-4 benzonitrile.
Fc = 54-58°C.
— puis CM 40847 A.
Fc = 210-213°C.

En utilisant des procédés de préparation analogues, on prépare les composés, selon l'invention, décrits dans le tableau ci-après. Ils sont caractérisés par leur point de fusion mesuré après recristallisation dans un mélange éthanol-éther.

TABLEAU 1

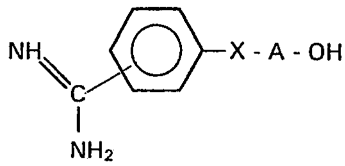

(I)

| N° produit | Position | X | A - OH | Préparé selon exemple | F. |
|---|---|---|---|---|---|
| SR 41613 A | p | — | $(CH_2)_4OH$ | 1 | 178-180°C |
| SR 41947 A | p | — | $(CH_2)_5OH$ | 1 | 210-215°C |
| SR 41149 A | p | — | $CH_2$-$CH(OH)nC_3H_7$ | 2 | 85-90°C |
| CM 40721 A | p | O | -$(CH_2)_3OH$ | 4 | 161-163°C |
| CM 40940 A | p | O | -$(CH_2)_5OH$ | 4 | 180-185°C |
| SR 41579 A | m | O | -$(CH_2)_4OH$ | 4 | 141-143°C |
| SR 41616 A | O | O | -$(CH_2)_4OH$ | 4 | 152-154°C |
| SR 42748 A | P | — | -$C(CH_3)_2CH_2OH$ | 3 | 181°C |

L'activité bactéricide des produits selon l'invention a été étudiée sur différentes souches par la méthode décrite ci-dessous:

Un inoculum bactérien est mis en contact avec différentes dilutions du produits à tester, et, ce, pendant un temps limité. A la fin du contact, une partie aliquote du mélange suspension bactérienne produit est déposée à la surface d'un milieu de culture gélosé contenant un neutralisant de l'activité antibactérienne du produit.

La concentration bactéricide retenue est la concentration minimale de produit à partir de laquelle les bactéries ne poussent plus. Cette concentration est exprimée en µg/ml.

Les souches bactériennes choisies pour l'étude sont:

1 - Escherichia Coli CNCM 54125;

2 - Klebsiella pneumoniae capsulée RO30;

3 - Pseudomonas aeruginosa CNCM A22;

4 - Streptococcus faecalis CNCM 5855;

5.- Staphylococcus aureus CNCM 53154.

La deuxième est entretenue sur milieu Worgel Fergusson, les autres sur Tryptic Soy Agar-Difco (TSA).

Après 24 h de culture à 37 °C, on récolte la pousse microbienne à l'aide de billes de verre et de 10 ml de diluant contenant 1 g de tryptone et 8,5 g de chlorure de sodium dans 1 000 ml d'eau distillée. On agite la suspension formée et on mesure au spectrophotomètre le pourcentage de transmission de la lumière à 620 nm:

- souche 1 : 70%;
- souche 2 : 80%;
- souche 3 : 70%;
- souche 4 : 60%;
- souche 5 : 60%.

L'inoculum bactérien correspond à une suspension au 1/20e de cette suspension bactérienne.

Des plaques comportant des cupules reçoivent différentes dilutions du produit à étudier. Ces dilutions du produit à étudier sont mises en contact avec les différentes suspensions bactériennes à l'aide d'un inoculateur à site multiple type Steers. Après 20 min de contact, des aliquotes sont transférées à l'aide de cet inoculateur à la surface d'un milieu gélosé (TSA) placé dans des boîtes de Petri, contenant un neutralisant de l'activité, à savoir 20 g de Lubrol W, 2,5 g de Tween 80 et 2,5 g de thiosulfate de sodium dans 1 000 ml de TSA (Difco). Un témoin de l'efficacité du neutralisant est réalisé pour chaque produit étudié en déposant à la surface du milieu de culture une aliquote de la dilution du produit à étudier. Après séchage, l'inoculum correspondant est déposé au même endroit. Un témoin inoculum est réalisé sur milieu gélosé avec et sans neutralisant. La lecture se fait après 48 h d'incubation à 37°C.

Les résultats sont rassemblés dans le tableau 2 ci-après.

TABLEAU 2

*Concentration minimale bactéricide (CMB) en µg/ml*

| N° du | Souches bactériennes | | | | |
|---|---|---|---|---|---|
| produit | 1 | 2 | 3 | 4 | 5 |
| SR 41613 A | 10 000 | 10 000 | 15 000 | 10 000 | 5 000 |
| SR 41946 A | 4 000 | 5 000 | 4 000 | 2 000 | 2 000 |
| SR 41149 A | 5 000 | 6 000 | 6 000 | 20 000 | 20 000 |
| CM 40721 A | 8 000 | 8 000 | 8 000 | 8 000 | 8 000 |

TABLEAU 2 (suite)

| N° du produit | Souches bactériennes 1 | 2 | 3 | 4 | 5 |
|---|---|---|---|---|---|
| CM 40847 A | 5 000 | 5 000 | 5 000 | 5 000 | 5 000 |
| SR 41616 A | 7 500 | 5 000 | 5 000 | 15 000 | 15 000 |
| CM 40940 A | 16 000 | 15 000 | 15 000 | 20 000 | 15 000 |
| SR 41579 A | 8 000 | 8 000 | 8 000 | 10 000 | 15 000 |
| SR 42748 A | 10 000 | 10 000 | ⩽5 000 | 20 000 | 10 000 |

Les résultats montrent que les produits selon l'invention présentent un niveau d'activité comparable sur l'ensemble des souches bactériennes testées.

Comparés à l'alcool phényléthylique, produit bactéricide employé tant comme antiseptique que comme conservateur, les produits selon l'invention ont un niveau moyen d'activité supérieur et sont par ailleurs solubles dans l'eau ce qui facilite beaucoup leur emploi particulièrement dans les formulations galéniques.

La tolérance des produits selon l'invention a été étudiée chez le cobaye. Les animaux sont tondus de part et d'autre de la ligne médiane du dos, la tonte est entretenue tous les 2 jours. Des lots de 6 reçoivent sur la zone tondue 0,2 ml d'une solution aqueuse ou alcoolique du produit selon l'invention. Lorsque les produits sont en solution alcoolique, un lot témoin d'animaux reçoit l'alcool sur un côté.

Pour l'étude de la tolérance cutanée préliminaire, le traitement est appliqué 1 fois par jour, 6 jours sur 7, durant 3 semaines. Les observateurs sur le plan cutané portent sur la présence d'érythème, d'éruption cutanée ou d'hyperkératose dont l'intensité est graduée selon un barême déterminé.

L'essai de sensibilisation cutané est réalisé sur les mêmes animaux après deux semaines de repos. Le traitement dure une semaine, il est identique au précédent. L'évaluation se fait sur les mêmes critères et d'après le même barême que celui utilisé pour la tolérance locale.

On a également recherché si les produits selon l'invention présentent un effet phototoxique ou photoallergique chez le cobaye. La technique mise en œuvre est celle de UNKOVIC J., MAZUE G. et GIRARD J., Sciences et Techniques de l'Animal de laboratoire vol. 8 (3), 149-160, (1983). C'est une adaptation des techniques décrites par HARBER L.C. et al, Arch. Dermatol., 1967, vol. 96, p. 646-656 et VINSON L.J. et al, J. Soc. Cosm. Chem., 1966, vol. 17, p. 123-130.

Aucun des produits étudiés n'a montré de mauvaise tolérance, d'effet sensibilisant ni d'effet phototoxique ou photoallergique chez le cobaye.

Les produits selon l'invention qui présentent une bonne activité antimicrobienne et qui sont bien tolérés pourront recevoir de multiples applications comme antiseptiques, conservateurs ou désinfectants dans les domaines humains, cosmétique et thérapeutiques, vétérinaire ou agroalimentaire.

En particulier, ils pourront être utilisés comme antiseptiques dans des préparations à visée thérapeutique , par exemple, dans le traitement de l'impétigo, l'acné, les dermatoses infectées, les plaies ouvertes infectées, les infections fermées telles que furoncles, panaris, gales impétiginisées etc. On peut également envisager une utilisation à visée préventive, par exemple, pour la préparation du champ chirurgical, la préparation des mains du chirurgien ou du personnel soignant.

En application vétérinaire, les produits selon l'invention peuvent être utilisés soit en tant qu'antiseptiques (par exemple dans la prévention des mammites) soit en tant que désinfectants (désinfection du matérial étables etc.) ainsi que dans le domaine agroalimentaire.

Enfin leur bonne tolérance et leur faible toxicité les autorisent à être utilisés comme conservateurs, non seulement dans le domaine de la pharmacie et de la cosmétologie, mais également en agroalimentaire.

Différentes formulations galéniques des produits selon l'invention peuvent être préparées en fonction de l'application choisie.

*Exemple 5*

*Préparation antiseptique liquide détergente moussante:*

| | | | |
|---|---|---|---|
| SR 41613 A | | 3 | g |
| Alkyldiméthylcarboxyméthylamine (solution à 30%) | | 15 | g |
| Tétracémate disodique | | 0,1 | g |
| Propylèneglycol | | 10 | g |
| Hydroxyde de sodium ou Acide lactique | qsp pH 5,8 | | |
| Eau purifiée | qsp | 100 | g |

*Exemple 6*

*Préparation antiseptiques liquide détergente moussante:*

| | | | |
|---|---|---|---|
| SR 41946 A | | 2 | g |
| Paraffine sulfonate de sodium | | 15 | g |
| Hydroxyde de sodium ou Acide lactique | qsp pH 5,2 | | |
| Eau purifiée | qsp | 100 | g |

*Exemple 7*

*Désinfectant pour surface inerte:*

| | | | |
|---|---|---|---|
| SR 41579 A | | 5 | g |
| Dodécyldiméthylcarboxydiméthylamine | | 20 | g |
| Tétracémate disodique | | 2 | g |
| Acide lactique | qsp pH 3,5 | | |
| Eau purifiée | qsp | 100 | g |

*Exemple 8*

*Soluté antiseptique alcoolique:*

| | | |
|---|---|---|
| SR 41946 A | | 2 g |
| Alkyldiméthylcarboxyméthylamine (solution à 30%) | | 0,5 g |
| Condensat d'oxyde d'éthylene et de propylèneglycol L 62 | | 1 g |
| Hydroxyde de sodium | qsp pH 6,5 | |
| Alcool éthylique à 70° | qsp | 100 g |

*Exemple 9*

Un produit selon l'invention peut être utilisé comme conservateur dans un shampooing:

| | | |
|---|---|---|
| Palmitate de potassium et d'acides aminés | | 20 g |
| Alkylsulfates de sodium | | 2 g |
| Diéthanolamide de coprah | | 5 g |
| Acétate de linolyle | | 0,200 g |
| SR 41946 A | | 0,150 g |
| Hydroxyde de sodium | qsp pH 7 | |
| Eau purifiée | qsp | 100 g |

*Exemple 10*

Un produit selon l'invention peut être utilisé comme conservateur dans une crème émulsion.

| | | |
|---|---|---|
| Huile de vaseline épaisse | | 6 g |
| Mélange d'alcool cétostéarylique et d'alcool cétostéarylique oxyméthylé | | 9 g |
| Phosphate monosodique anhydre | | 0,300 g |
| Tétracémate disodique | | 0,010 g |
| Vaseline | | 15 g |
| SR 41946 A | | 0,150 g |
| Acide phosphonique | qsp pH 4,5 | |
| Eau purifiée | qsp | 100 g |

*Exemple 11*

Un produit selon l'invention peut être utilisé comme conservateur dans une crème pour utilisation cosmétologique:

| | | |
|---|---|---|
| Collagène | | 0,500 g |
| Carboxypolyméthylène 934 | | 0,400 g |
| Lanoline hydrogénée | | 4 g |
| Perhydrosqualène | | 20 g |
| Monopalmitate de sorbitol polyoxyméthylène | | 2 g |
| SR 41946 A | | 0,150 g |
| Acide lactique ou Hydroxyde de sodium | qsp pH 6,5 | |
| Eau purifiée | qsp | 100 g |

**Revendications pour les Etats contractants:** BE, CH, DE, FR, GB, IT, LI, LU, NL, SE

1. Dérivés de la benzamidine répondant à la formule générale:

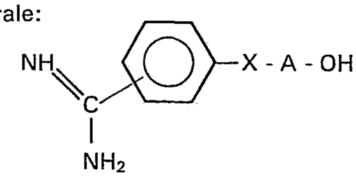

(I)

dans laquelle

A représente une chaîne alkyle droite ou ramifiée comprenant de 3 à 9 atomes de carbone;

X représente l'atome d'oxygène ou une liaison directe,

à la condition que, lorsque X représente une liaison directe, la benzamidine est située en para par rapport au groupement alcanol, ainsi que les sels pharmaceutiquement acceptables desdits dérivés.

2. Dérivés selon la revendication 1, caractérisés en ce que X est une liaison directe et A-OH est choisi parmi les groupements suivants: $-(CH_2)_3OH$; $-CH_2-CHOH-CH_2CH_3$; $-C(C_3H_7)_2CH_2OH$; $-(CH_2)_4OH$; $-(CH_2)_5OH$; $CH_2-CH(OH)nC_3H_7$; $-C(CH_3)_2CH_2OH$.

3. Dérivés selon la revendication 1, caractérisés en ce que X est l'oxygène et A-OH est choisi parmi $-(CH_2)_3OH$; $-(CH_2)_4OH$ et $-(CH_2)_5OH$.

4. Procédé pour l'obtention des dérivés de la benzamidine selon la revendication 1, caractérisé en ce qu'il consiste:

1) à soumettre à une hydrogénation catalytique le composé de formule IV:

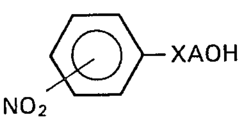

(IV)

dans laquelle X et A sont tels que définis dans la revendication 1, pour former les dérivés de l'aniline correspondants;

2) à ajouter auxdits dérivés de l'aniline du nitrite de sodium en milieu acide pour former les diazoniums correspondants;

3) à transformer lesdits diazoniums en benzonitriles par action du cyanure cuivreux;

4) à transformer le benzonitrile obtenu en composé de formule I et

5) éventuellement à transformer ledit composé obtenu en un sel pharmaceutiquement acceptable.

5. Composition pharmaceutique à usage externe présentant une activité antimicrobienne et désinfectante caractérisée en ce qu'elle contient une quantité efficace d'un composé selon la revendication 1.

6. Application des composés selon la revendication 1 à titre de conservateurs en pharmacie, cosmétologie et agroalimentaire.

7. Produits cosmétiques, caractérisés en ce qu'ils contiennent à titre de conservateurs, un composé de formule I selon la revendication 1.

8. Compositions désinfectantes pour surfaces inertes, caractérisées en ce qu'elles contiennent une quantité efficace d'un dérivé selon la revendication 1.

**Revendications pour l'Etat contractant:** AT

1. Procédé pour l'obtention de dérivés de la benzamidine répondant à la formule générale:

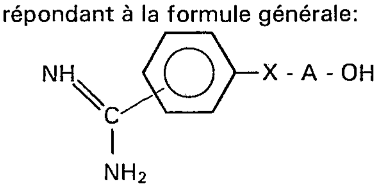

(I)

dans laquelle

A représente une chaîne alkyle droite ou ramifiée comprenant de 3 à 9 atomes de carbone;

X représente l'atome d'oxygène ou une liaison directe,

à la condition que, lorsque X représente une liaison directe, la benzamidine est située en para par rapport au groupement alcanol, ainsi que les sels pharmaceutiquement acceptables desdits dérivés, caractérisé en ce qu'il consiste:

1) à soumettre à une hydrogénation catalytique le composé de formule IV:

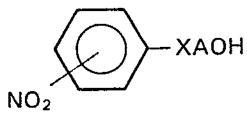
(IV)

dans laquelle X et A sont tels que définis ci-dessus, pour former les dérivés de l'aniline correspondants;

2) à ajouter auxdits dérivés de l'aniline du nitrite de sodium en milieu acide pour former les diazoniums correspondants;

3) à transformer lesdits diazoniums en benzonitriles par action du cyanure cuivreux;

4) à transformer le benzonitrile obtenu en composé de formule I et

5) éventuellement à transformer ledit composé obtenu en un sel pharmaceutiquement acceptable.

2. Procédé selon la revendication 1, caractérisé en ce que X est une liaison directe et A-OH est choisi parmi les groupements suivants:
$-(CH_2)_3OH$; $-CH_2-CHOH-CH_2CH_3$; $-C(C_3H_7)_2CH_2OH$; $-(CH_2)_4OH$; $-(CH_2)_5OH$; $CH_2-CH(OH)nC_3H_7$; $-C(CH_3)_2CH_2OH$.

3. Procédé selon la revendication 1, caractérisé en ce que X est l'oxygène et A-OH est choisi parmi les groupes $-(CH_2)_3OH$; $-(CH_2)_4OH$; et $-(CH_2)_5OH$.

**Patentansprüche für die Vertragsstaaten:**
BE, CH, DE, FR, GB, IT, LI, LU, NL, SE

1. Benzamidinderivate der allgemeinen Formel

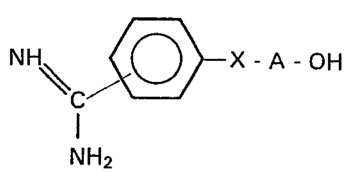
(I)

worin

A eine gerade oder verzweigte Alkylkette mit 3 bis 9 Kohlenstoffatomen bedeutet;

X ein Sauerstoffatom oder eine direkte Bindung darstellt,

mit der Massgabe, dass, wenn X eine direkte Bindung darstellt, sich das Benzamidin in bezug auf die Alkanolgruppe in der para-Position befindet, sowie die pharmazeutisch akzeptablen Salze dieser Derivate.

2. Derivate nach Anspruch 1, dadurch gekennzeichnet, dass X eine direkte Bindung ist und A-OH ausgewählt ist aus den folgenden Gruppen:
$-(CH_2)_3OH$; $-CH_2-CHOH-CH_2CH_3$; $-C(C_3H_7)_2CH_2OH$; $-(CH_2)_4OH$; $-(CH_2)_5OH$; $CH_2-CH(OH)_nC_3H_7$; $-C(CH_3)_2CH_2OH$.

3. Derivate nach Anspruch 1, dadurch gekennzeichnet, dass X für Sauerstoff steht und A-OH ausgewählt ist aus $-(CH_2)_3OH$; $-(CH_2)_4OH$ und $-(CH_2)_5OH$.

4. Verfahren zur Herstellung der Benzamidinderivate nach Anspruch 1, dadurch gekennzeichnet, dass

1) die Verbindung der Formel IV

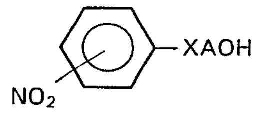
(IV)

worin X und A die in Anspruch 1 angegebenen Bedeutungen haben, einer katalytischen Hydrierung unterzogen wird, um die entsprechenden Anilinderivate zu bilden;

2) zu diesen Anilinderivaten Natriumnitrit in saurem Milieu zugegeben wird, um die entsprechenden Diazoniumverbindungen zu bilden;

3) die Diazoniumverbindungen durch Einwirken von Kupfer(I)-cyanid in Benzonitrile übergeführt werden;

4) das erhaltene Benzonitril in die Verbindung der Formel I übergeführt wird; und

5) die erhaltene Verbindung gegebenenfalls in ein pharmazeutisch akzeptables Salz übergeführt wird.

5. Pharmazeutische Zusammensetzung zur äusseren Anwendung mit antimikrobieller und desinfizierender Aktivität, dadurch gekennzeichnet, dass sie eine wirksame Menge einer Verbindung nach Anspruch 1 enthält.

6. Anwendung der Verbindungen nach Anspruch 1 als Konservierungsmittel in der Pharmazie, Kosmetik und Agrar-Nahrungsmittelindustrie.

7. Kosmetische Produkte, dadurch gekennzeichnet, dass sie als Konservierungsmittel eine Verbindung der Formel I nach Anspruch 1 enthalten.

8. Desinfektionszusammensetzungen für inerte Oberflächen, dadurch gekennzeichnet, dass sie eine wirksame Menge eines Derivats nach Anspruch 1 enthalten.

**Patentansprüche für den Vertragsstaat:** AT

1. Verfahren zur Herstellung von Benzamidinderivaten der allgemeinen Formel

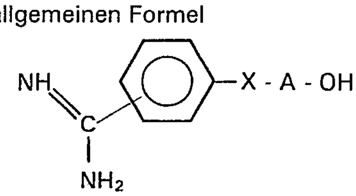
(I)

worin

A eine gerade oder verzweigte Alkylkette mit 3 bis 9 Kohlenstoffatomen bedeutet;

X ein Sauerstoffatom oder eine direkte Bindung darstellt,

mit der Massgabe, dass, wenn X eine direkte Bindung darstellt, sich das Benzamidin in bezug auf die Alkanolgruppe in der para-Position befindet, sowie die pharmazeutisch akzeptablen Salze dieser Derivate, dadurch gekennzeichnet, dass

1) die Verbindung der Formel IV

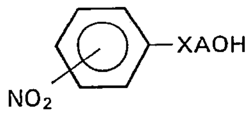

(IV)

worin X und A die in Anspruch 1 angegebenen Bedeutungen haben, einer katalytischen Hydrierung unterzogen wird, um die entsprechenden Anilinderivate zu bilden;

2) zu diesen Anilinderivaten Natriumnitrit in saurem Milieu zugegeben wird, um die entsprechenden Diazoniumverbindungen zu bilden;

3) die Diazoniumverbindungen durch Einwirken von Kupfer(I)-cyanid in Benzonitrile übergeführt werden;

4) das erhaltene Benzonitril in die Verbindung der Formel I übergeführt wird; und

5) die erhaltene Verbindung gegebenenfalls in ein pharmazeutisch akzeptables Salz übergeführt wird.

2. Verfahren nach Anspruch 1, dadurch gekennzeichnet, dass X eine direkte Bindung ist und A-OH ausgewählt ist aus den folgenden Gruppen:
$-(CH_2)_3OH$; $-CH_2-CHOH-CH_2CH_3$; $-C(C_3H_7)_2CH_2OH$; $-(CH_2)_4OH$; $-(CH_2)_5OH$; $CH_2-CH(OH)_nC_3H_7$; $-C(CH_3)_2CH_2OH$.

3. Verfahren nach Anspruch 1, dadurch gekennzeichnet, dass X für Sauerstoff steht und A-OH ausgewählt ist aus den Gruppen $-(CH_2)_3OH$; $-(CH_2)_4OH$ und $-(CH_2)_5OH$.

**Claims for the contracting states:**
BE, CH, DE, FR, GB, IT, LI, LU, NL, SE

1. Benzamidine derivatives corresponding to the general formula:

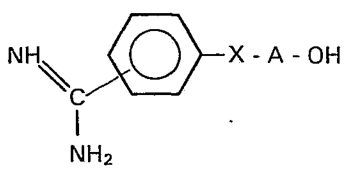

(I)

in which
A represents a linear or branched alkyl chain containing from 3 to 9 carbon atoms; and
X represents the oxygen atom or a direct bond,
with the proviso that if X represents a direct bond, the benzamidine is located in the para position relative to the alkanol group, and also the pharmaceutical acceptable salts of said derivatives.

2. Derivatives according to claim 1, characterized in that X is a direct bond and A-OH is chosen from the followings groups:
$-(CH_2)_3OH$; $-CH_2-CHOH-CH_2CH_3$; $-C(C_3H_7)_2CH_2OH$; $-(CH_2)_4OH$; $-(CH_2)_5OH$; $CH_2-CH(OH)nC_3H_7$; $-C(CH_3)_2CH_2OH$.

3. Derivatives according to claim 1, characterized in that X is oxygen and A-OH is chosen from $-(CH_2)_3OH$; $-(CH_2)_4OH$ and $-(CH_2)_5OH$.

4. Process for the preparation of the benzamidine derivatives according to claim 1, characterized in that it consists in:

1) subjecting the compound of the formula IV:

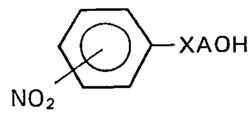

(IV)

in which X and A are as defined in claim 1, to a catalytic hydrogenation in order to form the corresponding aniline derivatives;

2) adding sodium nitrite in an acid medium to the said aniline derivatives in order to form the corresponding diazonium compounds;

3) converting the said diazonium compounds to benzonitriles by reaction with cuprous cyanide;

4) converting the resulting benzonitrile to the compound of the formula I and

5) if possibly, converting the said resulting compound to a pharmaceutically acceptable salt.

5. A pharmaceutical composition for external use, having an antimicrobial and disinfectant activity, characterized in that it contains an effective quantity of a compound according to claim 1.

6. The use of the compounds according to claim 1 as preservatives in pharmacy, cosmetology and food crops.

7. Cosmetic products characterized in that they contain, as preservatives, a compound of the formula I according to claim 1.

8. Disinfectant composition for inert surfaces, characterized in that they contain an effective quantity of a derivative according to claim 1.

**Claims for the contracting state:** AT

1. Process for the preparation of benzamidine derivatives corresponding to the general formula:

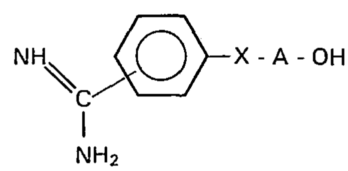

(I)

in which
A represents a linear or branched alkyl chain containing from 3 to 9 carbon atoms;
X represents the oxygen atom or a direct bond,
with the proviso that if X represents a direct bond, the benzamidine is located in the para position relative to the alkanol group, and also the pharmaceutical acceptable salts of said derivatives, characterized in that it consists in:

1) subjecting the compound of the formula IV:

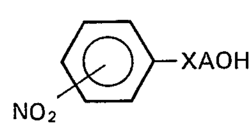

(IV)

in which X and A are as defined above, to a catalytic hydrogenation in order to form the corresponding aniline derivatives;

2) adding sodium nitrite in an acid medium to the

said aniline derivatives in order to form the corresponding diazonium compounds;

3) converting the said diazonium compounds to benzonitriles by reaction with cuprous cyanide;

4) converting the resulting benzonitrile to the compound of the formula I and

5) if possibly, converting the said resulting compound to a pharmaceutically acceptable salt.

2. Derivatives according to claim 1, characterized in that X is a direct bond and A-OH is chosen from the followings groups:
$-(CH_2)_3OH$; $-CH_2-CHOH-CH_2CH_3$; $-C(C_3H_7)_2CH_2OH$; $-(CH_2)_4OH$; $-(CH_2)_5OH$; $CH_2-CH(OH)nC_3H_7$; $-C(CH_3)_2CH_2OH$.

3. Derivatives according to claim 1, characterized in that X is oxygen and A-OH is chosen from $-(CH_2)_3OH$; $-(CH_2)_4OH$ and $-(CH_2)_5OH$ groups.